# EUROPEAN PATENT APPLICATION

(11) **EP 2 316 510 A2**
(43) Date of publication of application: **04.05.2011**
(21) Application number: 10188054.0
(22) Date of filing: 19.10.2010
(51) Int. Cl.: A61M 5/32, A61M 5/00

(54) **Shield apparatuses and methods for storing syringe assemblies and needle assemblies**

(30) Priority: 19.10.2009 US 252962 P
(71) Applicant: Terumo Medical Corporation, Somerset, New Jersey 08873 (US)
(72) Inventor: Powers, Thomas, Newtown Pennsylvania 18940 (US); Dowdell, Ralph, Trenton New Jersey 08611 (US); Hidalgo, Craig, Langhorne Pennsylvania 19047 (US); Bredael, Gary, Langhorne Pennsylvania 19053 (US); Mathews, David, Joppa Massachusetts 21085 (US); Voellmicke, Craig, New York New York 10023 (US)
(74) Representative: Jenkins, Peter David

(57) **Abstract**

The present invention is directed to shield apparatuses configured to hold syringe assemblies in a manner that preserves their ease of use and convenience. In some embodiments, the syringe assemblies are detachable, and the shield apparatuses provide for convenient interchanging of the needle assemblies, as well as methods for administering injections to a patient.

## Description

### CROSS REFERENCE TO RELATED APPLICATIONS

This application claims benefit of U.S. Provisional Application No. 61/252,962 filed October 19, 2009, hereby incorporated by reference in its entirety.

### BACKGROUND OF THE INVENTION

The present invention is related to the field of hypodermic syringes and needles, and particularly to syringe assemblies having detachable needles, low dead space and a bevel indicator; as well as shield apparatuses for protecting and storing such syringe assemblies; as well as kits comprising syringes and interchangeable needle assemblies; all of which increase the ease and convenience of use for medical staff and patients that use such syringes to provide multiple injections over a short period of time.

Hypodermic syringes are commonly used by doctors, nurses and other medical staff (including staff trained in cosmetic procedures) for various procedures that require injection of medication and other medical or cosmetic substances into patients' bodies. Such syringes are also commonly used by certain patients, as for example, diabetics who may self-inject insulin.

In certain situations where a patient must be given multiple injections, for example for allergy injections, injections of insulin for diabetics or cosmetic applications, it is desirable to avoid dulling of a hypodermic needle over multiple uses, as dulling can lead to decreased accuracy in drug delivery and patient discomfort. Further, in view of recent industry-wide attention to minimizing the environmental impact of medical waste, it is desirable to provide syringe assemblies that permit convenient use and interchangeability of needles with syringes, such as providing a single syringe that can be used multiple times on a single patient, where a used needle attached to the barrel of a syringe can quickly and easily be replaced with a fresh and sterile needle.

In general, an injection is administered in a manner including a "loading" step - that is, a step wherein the administrator or patient obtains a fully assembled but empty syringe assembly with the plunger fully depressed into the syringe barrel, then "loads" the syringe assembly by inserting the needle portion into a source of fluid (for example, a container that holds a reservoir of the fluid to be injected into the patient). This step is followed by the administrator or patient's pulling out the plunger by sliding it along the longitudinal axis of the barrel in a proximal direction, engaging a vacuum force to load the syringe with a predetermined amount of fluid. The needle is then withdrawn from the container, fully loaded and ready to inject into the patient. Repeated loading can also dull the needle.

Small needles have not traditionally been commonly used, but are gaining in popularity. For example, needles as small as 29-30 gauge are often used to inject insulin, and even needles as small as 31-33 gauge are now becoming more widely used (the "gauge" of a needle is a way of denoting the outer diameter of the needle, and a higher gauge indicates a small needle). Some uses for lower gauge needles include injecting insulin, administering allergy injections and for cosmetic applications such as the administration of Botox®. Further, allergy injections and Botox® injections generally require multiple injections to a patient in a single treatment. Thus, it may be desirable to use smaller needles for comfort and convenience for the patient. However, such needles are generally fragile because of their smaller size, and tend to be undesirable for loading, as their small circumference leads to slow loading and an increased risk of breakage during the handling of the syringe before injection. Moreover, multiple injections and the need to insert the needle through a barrier the top of a bottle or vial of sterile fluid as part of the loading step may all lead to faster dulling of the needle, causing further discomfort to the patient. Thus, the needles that are desirable for certain injections because of their small size are at the same time not so desirable for loading such syringes before injection into the patient. Yet, in the syringe and needle assemblies in the current art, users generally do not have the option of using one needle to load the syringe and another to inject the fluid into the patient. While a large needle may be fast to load, it may cause greater physical pain to the patient during injection, and such pain is multiplied with multiple injections. On the other hand, a small needle can be difficult to load. Thus, a user is often left with two undesirable alternatives.

In the situations where repeated loadings and injections on a single patient are desirable, there are other disadvantages. Because many small syringe assemblies do not have detachable needles, a user may choose to use a fresh syringe for each injection to avoid dulling the needle. Discarding each syringe can be expensive and wasteful. Further, repeated injections over time may incrementally increase wasted fluid, as each injection will result in some fluid that is caught in the "dead space" in the syringe assembly rather than injected into the patient. This can be very expensive in the case of high-cost cosmetic fluid preparations such as Botox®.

Still further, many hypodermic needles are designed with a bevel tip that is cut at an angle, to facilitate smooth injection. The angle of such tip is known as the "bevel angle." Most bevel tips are cut as a "regular" bevel, which makes the tip appropriate for intramuscular delivery. However, other types of bevels include "short" bevels and "intradermal" bevels (all are based on the angle of the tip). To minimize a patient's discomfort, it is generally desirable to orient a needle so that the sharpest and farthest extending point (the "needle point"), rather than the part of the point that is closest to the hub (the "needle heel") breaks the patient's skin first. In the case of larger (lower gauge) needles known in the art, the bevel angle is visible to the user's naked eye, and therefore this is fairly easy to do. However, in the case of extremely small needles (higher gauges of higher than 25), such as those used for multiple injections as discussed herein, the bevel tip may be very small and difficult or impossible to see.

Thus, a need exists for improved syringes that have detachable and interchangeable needles, such that a used needle may be easily removed from a syringe and replaced with a fresh needle; and that also minimize dead space after injection to preserve as much fluid as possible; as well as kits comprising such syringes and needles that permit easy detachment and interchanging of such parts for users of multiple needles; as well as shield apparatuses for protecting such syringe assemblies before and after use.

### SUMMARY OF THE INVENTION

In certain embodiments, the present invention is directed to a shield apparatus for storing a needle assembly, the needle assembly comprising a needle fixed to a hub and having a needle point;
wherein the shield apparatus comprises a housing having an exterior wall and an interior wall defining an interior chamber that encloses the needle point;
wherein the shield apparatus is capable of freely standing on a horizontal plane, such that when the shield apparatus stores a syringe assembly, the needle point is facing substantially vertically downward.

In other embodiments, the present invention is directed to a shield apparatus for needle assemblies, the shield apparatus comprising:
(a) a housing having an exterior wall and an interior wall defining two chambers, each of the two chambers having a length and a width, and each of the two chambers having an open end and a closed end and configured to hold a needle assembly within.

In other embodiments, the present invention is directed to a syringe assembly comprising:
(a) a cylindrical barrel; and
(b) a needle assembly detachably fixed to the distal end of the cylindrical barrel;
wherein at least a portion of the needle assembly is contained within a shield apparatus.

In other embodiments, the present invention is directed to a method of interchanging a first needle assembly and a second needle assembly from a syringe assembly, the method comprising the steps of:
(a) obtaining a syringe assembly including a first needle assembly, wherein the first needle assembly is detachably fixed to the syringe assembly;
(b) inserting the syringe assembly into a first shield apparatus, needle assembly side first;
(c) detaching the first needle assembly from the syringe assembly;
(d) inserting the syringe assembly into a second shield apparatus, the second shield apparatus containing a second needle assembly; and
(e) attaching the second needle assembly to the syringe assembly.

In other embodiments, the present invention is directed to a method of administering an injection to a patient, the method comprising the steps of:
(a) obtaining a syringe assembly including a first needle assembly, the first needle assembly including a needle having a gauge of 25 or lower;
(b) loading the syringe assembly with a predetermined amount of fluid;
(c) inserting the syringe assembly into a first shield apparatus, needle assembly side first;
(d) detaching the first needle assembly from the syringe assembly;
(e) inserting the syringe assembly into a second shield apparatus, the second shield apparatus containing a second needle assembly, the second needle assembly including a needle having a gauge of higher than 25; and
(f) attaching the second needle assembly to the syringe assembly.

### BRIEF DESCRIPTION OF THE DRAWINGS

**Fig. 1** shows a shield apparatus in accordance with one embodiment of the present invention holding a syringe assembly; as well as a separated shield assembly and a separated needle assembly.

**Fig. 2** shows an internal view of the needle assembly portion of a syringe assembly as it is held within a shield apparatus in accordance with one embodiment of the present invention.

**Fig. 3** shows a close up internal view of the entry of a needle assembly into a shield apparatus in accordance with one embodiment of the present invention.

**Figs. 4a** and **4b** show external and internal views, respectively, of a shield apparatus holding a syringe assembly in accordance with one embodiment of the present invention.

**Fig. 5** shows a cross sectional internal view of a shield apparatus in accordance with one embodiment of the present invention.

**Fig. 6** shows a close up internal cross sectional view of a shield apparatus in accordance with one embodiment of the present invention, comprising two chambers configured to hold two needle assemblies of different size.

**Fig. 7** shows an external view of a shield apparatus holding a syringe assembly in accordance with one embodiment of the present invention.

**Fig. 8** shows an internal cross sectional view of a shield apparatus in accordance with another embodiment of the invention.

### DETAILED DESCRIPTION OF THE INVENTION

The shield apparatuses of the present invention have been found to be particularly useful for applications directed to unique types of syringe assemblies - specifically, syringe assemblies that exhibit the characteristics of low dead space, detachability of the needle portion, and in certain embodiments, a bevel indicator to assist a user in guiding the needle into the patient's body at the optimal point. All of these are attributes of certain embodiments of the syringe assemblies that can be held within the shield apparatuses of the present invention, and all will be discussed below. Desirable syringe assemblies are discussed in great detail in copending U.S. Application No._, filed on the same date as the present application and entitled "Syringe Assemblies Having Detachable Needle Assemblies and Low Dead Space" the disclosure of which is hereby incorporated by reference in its entirety. Kits comprising such assemblies are discussed in great detail in copending U.S. Application No._, filed on the same date as the present application and entitled, "Kits Comprising Syringe Assemblies" the disclosure of which is hereby incorporated by reference in its entirety.

The syringe assemblies discussed therein are particularly useful for the injection of medical or cosmetic fluid to treat conditions such as, but not limited to, allergies, hypohydrosis, muscle twitches, crossed eyes, cerebral palsy or the like, that require multiple injections along multiple sites on a patient's face or body in a single procedure or series of procedures. As used herein, the terms "medical or cosmetic fluid" refer to any fluid material that is desired to be delivered to a patient's body by injection using a syringe assembly, for treating a medical condition or providing a cosmetic benefit. In certain embodiments, the medical or cosmetic fluid may comprise, but is not limited to, allergenic and allergy-triggering compositions such as dander, or cosmetic compositions such as botulism toxin (in the case of cosmetic fluids such as Botox®) and muscle relaxants and the like.

### Low Dead Space

The issue of dead space is an ongoing concern in the field of syringes and other surgical instruments that deliver fluids. A primary concern is that whenever currently used syringes are fully deployed, liquid remains in the syringe, generally in the distal end of the barrel that may comprise a conical or cross-sectionally trapezoidal shape, as well as in the length of the needle itself. This liquid is lost after deployment of the syringe assembly, as there is no way to extract it from the syringe or force it from the barrel through the needle. For example, the distal end of the plunger may be substantially flat and at a substantially perpendicular angle to the longitudinal axis of the syringe barrel; the corresponding roof of the barrel may also be substantially flat in the same way, such that when the two meet, an optimal amount of fluid may be forced out of the barrel and through the needle.

Attempts have been made to both minimize dead space and provide detachable needle assemblies. See, for example, U.S. Patent No. 5,782,803 to Jentzen and U.S. Patent Publication No. 2008/0033347 to D'Arrigo. However, these and other references in the art address syringes more traditionally used that have larger needles (generally gauges of far lower than 29) with a capacity of 3 mL and larger. The interchangeability and dead space of small gauge needles has not been adequately addressed by prior attempts. As discussed herein, small gauge needles (for example, 29-33 gauge needles and even smaller) present unique challenges in the art, including but not limited to their fragility and difficulty to detach due to their small size.

It has been discovered that minimization of dead space can be achieved via the design of the plunger and syringe barrel configurations. Specifically, the interior of the barrel is configured to receive a plunger, and the plunger has a distal end (which is inserted into the barrel) and a proximal end, which in certain embodiments, comprises a plunger handle to facilitate the user's sliding the plunger along the longitudinal axis of the barrel. The cylindrical barrel comprises an interior cavity, and at the distal portion of the cavity is an interior distal surface, which the plunger touches when fully deployed - that is, fully slid into the barrel such that it can slide no farther in. This interior distal surface of the barrel that the distal end of the plunger ultimately touches when fully deployed, and which represents the farthest surface to which the plunger can travel into the barrel, is referred to herein as the "roof" of the barrel.

While at least a portion of the roof of the barrel is a closed surface in order to receive the plunger, the roof of the barrel will, in certain embodiments, comprise at least one opening that permits fluid communication of the interior of the barrel with the needle assembly, thus permitting injection of the fluid from the barrel into the body of a patient via the hollow lumen of the needle when the user depresses the plunger toward the roof. The greater the surface area of contact between the distal end of the plunger (when fully deployed) and the roof of the barrel, the less dead space results. In certain embodiments of the present invention, the distal end of the plunger and the roof both have shapes that correspond to and complement each other - that is, the distal end of the plunger has a shape that corresponds to an opposite and complementary shape on the roof of the distal portion of the interior space, such that when the plunger is fully deployed within the cylindrical barrel, the distal end of the plunger and the roof of the distal portion of the interior space mate at an interface to form a seal that forces substantially all of the fluid away from the interface and through the opening through which the interior of the barrel communicates with the needle assembly. The tighter this seal, the more dead space can be minimized and the more fluid can be forced out of the syringe barrel.

In certain embodiments, the shield apparatuses of the present invention hold a syringe assembly configured such that less than about 5% of the fluid originally loaded into the barrel is lost to dead space when the plunger of the syringe assembly is fully deployed. In various embodiments, this value is less than about 4%, less than about 3%, less than about 2%, or less than about 1%. In certain embodiments, the amount of fluid lost is less than about 0.5 mL, less than about 0.4 mL, less than about 0.1 mL, less than about 0.05 mL or less than about 0.01 mL.

### Detachable Needle Assembly

In various embodiments, the shield apparatuses of the present invention hold a syringe assembly that comprises a detachable needle assembly. As used herein, "detachable" means that a user can both attach the needle assembly to, and remove the needle assembly from, the distal end of the barrel without damaging either the needle assembly or the barrel or the connection between them.

Thus, in certain embodiments, the syringe assemblies that are held by the shield apparatuses of the present invention comprise two major components: first, a cylindrical barrel having disposed therein a plunger (herein also referred to as the "barrel," "cylindrical barrel" or "syringe barrel"); and second, a needle assembly comprising a hub fixed to a needle. In certain embodiments, the needle assembly is detachably fixed to the cylindrical barrel via any of the following mechanisms:
(a) a threaded portion on the distal end of the barrel that mates with a threaded portion of the syringe assembly ("Threading Connection");
(b) a portion on the distal end of the barrel that fits with a corresponding and complementary portion on the syringe assembly by a snapping together ("Snap Connection"); or
(c) male and female portions that are capable of being engaged together and connected by twisting ("Binary Connection").

In addition to being set forth in detail in copending U.S. Application No. _, filed on the same date as the present application and entitled "Syringe Assemblies Having Detachable Needle Assemblies and Low Dead Space," these mechanisms will be further discussed in greater detail herein.

A. Threading Connection

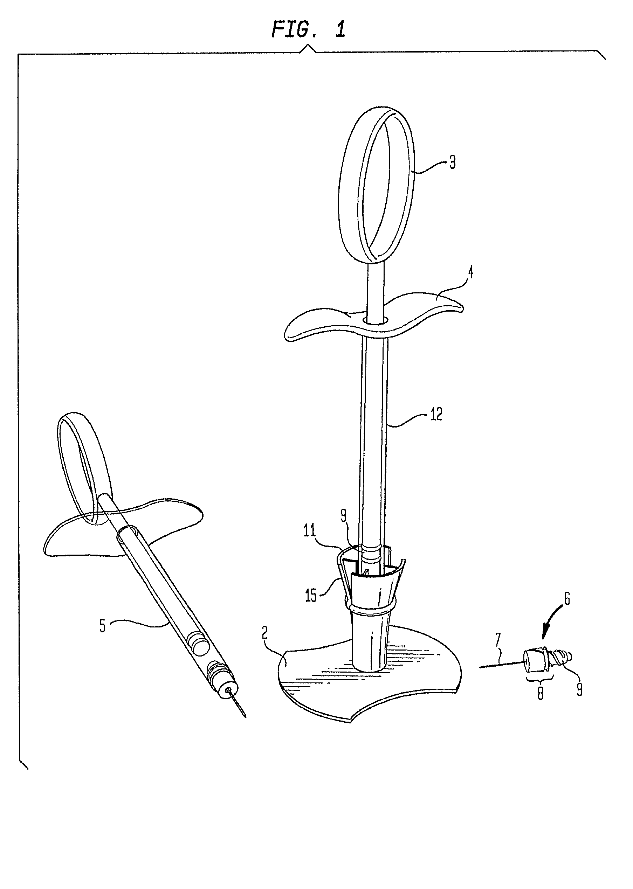

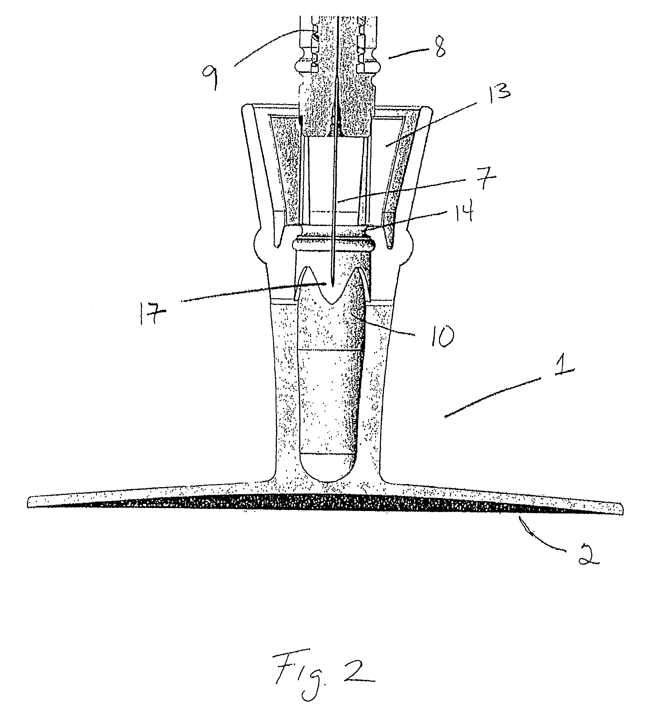

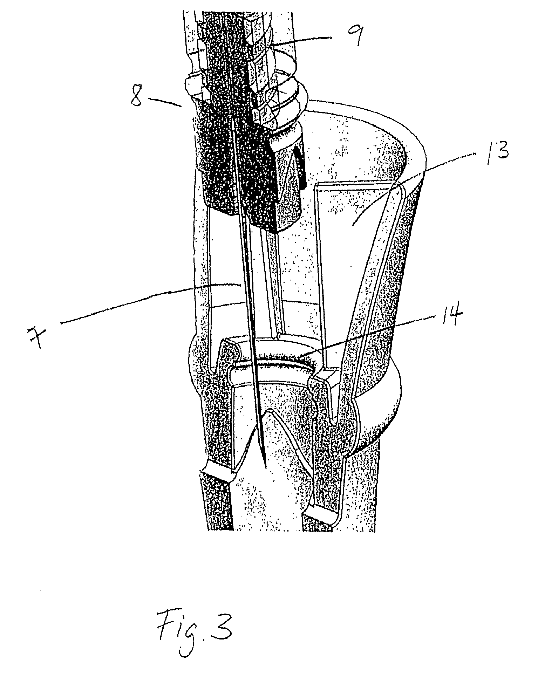

In certain embodiments, the connection between the syringe and the needle may be made by one or more threads on the needle assembly, where the threads correspond to and mate with corresponding threads on the distal end of the syringe assembly at the point of attachment, as with a screw. Examples of a threading connection can be seen, e.g., in **Fig. 2** and **Fig. 3****.** In **Fig. 3****,** the threads 9 can be seen disposed within the hub 8. The needle assembly comprises the hub 8 fixed to the needle 7. When the needle assembly and the distal end of the syringe assembly are fully threaded together, the needle 7 is in seamless fluid communication with the interior of the barrel 12 (not pictured in **Fig. 3****,** but visible in **Fig. 1**). In certain embodiments, the threading mechanisms connecting the needles to the syringes may further comprise one or more attachments that serve the purpose of locking the two together after threading, such that movement or pushing of the syringe or needle does not cause the needle to "walk off" the syringe - that is, that inadvertent unthreading does not occur. Such a locking mechanism may be in the form of a snap, a hook, a clamp flange or other similar mechanism, as well as adhesive or a magnetic attachment.

B. "Snap" Connection

In certain embodiments, the connection between the syringe assembly's barrel and the needle assembly may be made with corresponding mating parts on the needle (or attachment thereto) and the syringe, for example, "male" and "female" parts that mate together by a "click" or "snap" mechanism. In such embodiments, either of the needle assembly or the distal end of the barrel may be configured to have a slightly larger diameter than the other, such that one of the two can fit over the other. In certain embodiments, both may be of substantially the same diameter, but either of the needle assembly or the distal end of the barrel may be configured to deform and then pop into place in a manner that the two are not easily separated during use of the syringe assembly and insertion of the needle assembly portion into the shield apparatuses of the present invention; in certain embodiments, may be locked or joined tightly into place.

C. Binary Connection

In various embodiments, the connection is of a binary nature - that is, the corresponding ends of the syringe or needle (or attachment thereto) have locking portions (for example, a flange or a lip) that can be mated by inserting one portion into a corresponding and complementary portion of another and then making a "twist" to lock the two in place. Generally, the "twist" action may be less than a full 360 degree twist-that is, a fraction of the outer circumference of the locking portions. In various embodiments, the "twist" action can be accomplished by twisting to an amount of about 45 to about 270 degrees, about 50 to about 180 degrees, about 55 to about 120 degrees or about 45, about 90, about 120, about 180 or about 270 degrees. By "binary" it is meant that the corresponding parts can be configured in two possible ways - locked and unlocked or fixed and unfixed or attached and unattached.

A shield assembly that holds a syringe assembly according to any of these embodiments of the invention may comprise, as stated above, a configuration wherein a user can easily detach and interchange needles by inserting the end of a used needle into a chamber, giving the syringe a fractional turn (in various embodiments, a quarter turn, a half turn or a turn of about 45 degrees, about 90 degrees, about 120, about 180 or about 270 degrees), thereby detaching the used needle. The user can then leave the needle assembly portion held within the chamber and insert the remainder of the syringe assembly into a different chamber, give a similar turn and engage a fresh needle that is ready for use once it is pulled out of the chamber.

### Bevel Indicator

As discussed herein, many needles comprise a beveled tip, such that the tip is at an angle. Bevel tips are highly desirable, and in certain embodiments, it is desirable to inject the needle with its bevel point entering the patient first, to minimize discomfort. While for larger needles (for example, needles having 18-25 gauge) the user can make this determination with the naked eye, this is almost impossible to do with small needles. Thus, in certain embodiments of the present invention, the needle hub, or any other point along the needle or syringe assembly, may comprise a bevel indicator. In particular, where the syringe assembly is detachable from the barrel, in certain embodiments the bevel indicator is present at least in part on the hub, and may be a line or a dot or other easily discernible indicator, and may be colored in a manner that its contrast with the color of the hub, and/or raised or recessed or having a texture in a manner that its contrast with the texture of the hub and thus visible or otherwise discernible to the user. In this manner, the user can use the bevel indicator to determine the best direction from which to inject the needle into the patient's body.

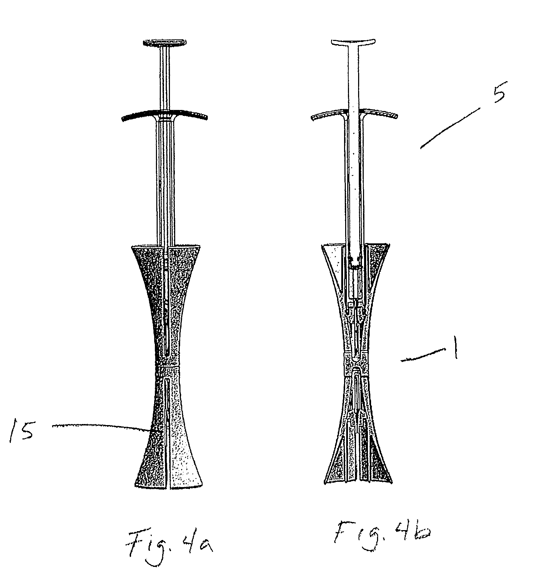

Similarly, in certain embodiments, the shield apparatuses of the present invention may exhibit markings (for example, lines, dots, raised areas, textured areas) or other indicia that indicate to a user, among other things: the size of the needle held therein, the status of the needle assembly (whether "fresh" or used); the direction or configuration of the needle point; or any other identifying information signifying the size (gauge) or type of needle. For example, as can be seen in **Fig. 4a**, a slit 15 is present on a portion of the longitudinal axis of the shield apparatus in that embodiment. One or more of such slits 15 make it possible for a user to see the interior of the shield apparatus, and may be useful in displaying color coded information. They are also useful in providing flexibility for the shield apparatus, as the slits allow it to expand and contract as necessary to receive the syringes therein.

As a further example, each chamber may contain a mechanical trigger, such that upon insertion of a used needle, a color-coded panel or other indicator (e.g., a word, picture or shape) is displayed on the shield apparatus, triggered by mechanical action. This can serve the purpose of notifying the user that the needle contained therein has been used and should not be touched again. It can also indicate to a user, in the case of a multiple-chamber shield apparatus, when it is time to discard the shield apparatus and obtain a new one.

### Using the Shield Apparatus

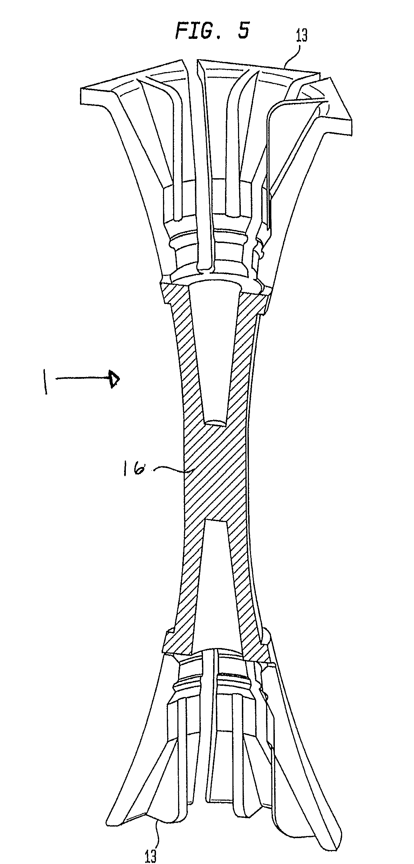

Thus, in certain embodiments, the present invention is directed to a shield apparatus comprising one or more chambers, at least one of the chambers enclosing a needle. As can be seen from **Fig. 1**, in certain embodiments, the shield apparatus 1 has a substantially flat face 2 that enables a user to stand the syringe apparatus up on its end, protected by the shield apparatus. In other embodiments, as seen in **Fig. 5**, the bottom portion is not a continuous flat face, but rather comprises a substantially flat plane formed by the edges of the engaging fins 13, as will be discussed further in the present disclosure. A user can easily detach the syringe apparatus from the shield apparatus by grasping the syringe flange 4 to disengage the syringe apparatus from the shield apparatus. After delivering the injection the patient, the syringe apparatus can similarly be reengaged with the shield apparatus, thus eliminating any risk of accidental contact by the user and the used needle. A syringe assembly separate from the shield apparatus 5 and a separate needle assembly 6 (which includes the needle hub and the needle) can also be seen in **Fig. 1**.

An internal view of the shield apparatus 1 of the present invention can be seen in **Fig. 2**. Here, the needle 7 is on its way to being fully inserted within the shield apparatus 1. The needle point 17 should be completely disposed within the chamber 10. That is, the chamber 10 encloses the needle point such that there is no danger of touching it when the shield apparatus is handled. In certain embodiments, when fully inserted, the hub 8 of the syringe assembly will engage onto an engaging fin 13 and engaging lip 14 of the shield apparatus. The engaging fin and engaging lip of the shield apparatus may lie at any point from the top of the shield apparatus to a point in the interior cavity of the apparatus. As can be seen in **Fig. 2** and **Fig. 3**, there may be one or more engaging fins 13 disposed around the interior cavity of the shield apparatus. The engaging fins 13 serve the dual purpose of ensuring that the barrel slides correctly into the shield apparatus, and in the case of a shield apparatus having two chambers, may additionally provide a standing support (as shown in **Fig. 5**).

As for the engaging lip 14, this may be present as an unbroken lip around the interior perimeter of the cavity, or in certain embodiments may be present as a structure that covers only part of the interior perimeter. The engaging lip will engage the hub and hold it, in part or in whole, within the shield apparatus. In the shield apparatus shown in **Fig. 2**, the syringe assembly is detachably fixed to the distal end of the syringe barrel (not shown) via a threading connection (threads 9 may be present on both the distal end of the barrel and the needle assembly 6). After insertion of the needle assembly into the chamber 10 of the shield apparatus and "catching" it on the engaging lip, the user can either leave the syringe apparatus as-is for storage or discarding, or can choose to detach the syringe barrel from the needle assembly and remove it, leaving the needle assembly protected and held in place within the shield apparatus. The engaging lip may have flexible portions (that is, portions that give slightly when pulled in a horizontal direction, but pop back into place once placed around an elongated item such as a syringe barrel or hub of a needle assembly) to engage corresponding edges on the end of the syringe and lock the needle assembly into place.

In another embodiment, the shield assemblies of the present invention may comprise two or more chambers, for storage of multiple needle assemblies. The chambers may be configured such that the needle points on the needle assemblies held therein face the closed end of each chamber. This is shown in **Figs. 4a** and **4b**, which show external and internal views, respectively, of a shield apparatus in accordance with the present invention. A user may use the syringe assembly 5, then insert it into one chamber 10 of the shield apparatus, detach the needle assembly from the remainder of the syringe assembly, and remove all but the needle assembly, leaving the needle assembly held in the chamber. The remainder of the syringe assembly can be subsequently attached to a fresh needle assembly for reuse, or discarded. In another embodiment, after all remaining needle assemblies in the shield apparatus are used, the user may insert the final needle assembly into the shield apparatus, but this time leave the syringe apparatus attached to it (rather than detached). The user may then discard the entire combination with the used needle safely enclosed within the shield apparatuses, without fear that the syringe and used needle will become separated.

For example, a patient may have a desire to complete several Botox® injections, allergy injections or insulin injections quickly, or a patient may be suffering pain or discomfort due to muscle spasms or another condition necessitating a series of injections rapidly administered (for example, allergies or cerebral palsy). In such situations, a patient may be impatient, agitated or emotional (this is especially true if the patient is a child), and anything that will streamline the process of injecting, reloading and re-injecting is likely to be desirable from the standpoint of patient comfort. In particular, embodiments of the present invention provide an easy way for a user to go through the entire process of unwrapping a syringe from its packaging, loading the drug (for embodiments wherein the drug is not pre-loaded into the syringe), preparing the appropriate needle for injection, injecting the drug, detaching the needle, discarding the used needle and re-loading a fresh needle as necessary, all with minimal effort.

In certain embodiments of the present invention, the shield apparatuses are configured such that they capable of freely standing on a horizontal plane, without assistance or intervention, such that when any portion of the needle assembly is held within the shield apparatus, the needle point is facing substantially vertically downward (as shown in **Fig. 1****,** **Figs. 4a** and **4b**). In various embodiments, any of the above steps can be accomplished using a single hand, thus decreasing the chances of accidental puncturing with a used needle and maximizing the user's ability to accomplish the desired results without additional help.

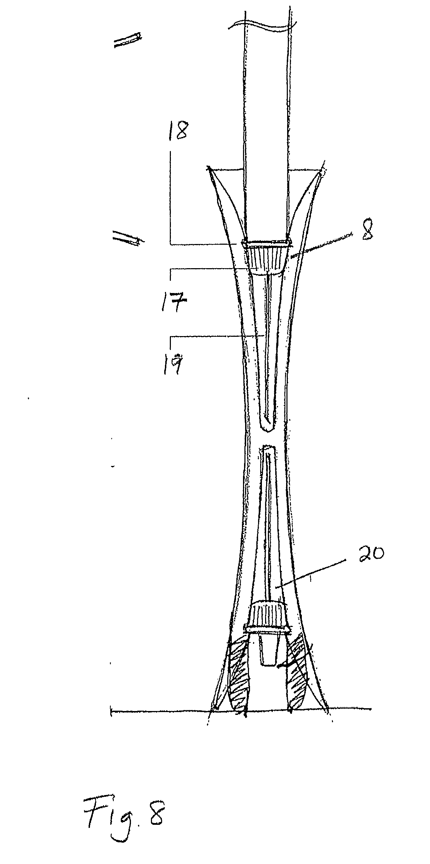

Exemplary shield apparatuses in accordance with the present invention can be seen, for example, in **Figs. 4a** and **4b****,** **Fig. 5** and **Fig. 8**, which all depict double-ended shield apparatuses that can stand on one end and may hold more than one needle assembly. In the embodiment shown in **Fig. 8**, two needle assemblies are present in a shield apparatus. The first needle assembly 19, on top, is connected to a syringe barrel. The second needle assembly 20, on the bottom, is separately held within the chamber on the bottom. The hub 8 of each syringe assembly shown comprises one or more vertical ribs 17. These can prevent the hub from spinning while store inside the shield apparatus. This can be especially important in the embodiments where the connection between the needle assembly and the syringe barrel is a threading connection. The hub 8 may further comprise a hub ring 18, which snaps into a corresponding slot in the interior of the shield apparatus, further holding the needle assembly in place.

In certain embodiments, the chambers may be of substantially equal size or may be of different size; for example, one chamber may be capable of holding a smaller-gauge (larger) needle while another may be capable of holding a larger-gauge (smaller) needle. In this way a user may load the syringe assembly with the larger needle, and then insert the larger needle assembly into the larger chamber, detach it and then attach the smaller needle assembly from the other side of the shield apparatus. Lower gauge (larger) needles are more desirable for loading, as they can load more fluid in a faster amount of time, with less danger of breaking or dulling the needle. In various embodiments, the first (loading) needle may be a lower gauge (larger) needle; for example, 25 gauge or lower, 18 to 25 gauge, or 18, 20 or 22 gauge. In various embodiments, the second (injecting) needle may be a higher gauge (smaller) needle; for example, higher than 25 gauge, 26-34 gauge, or 29, 30, 31, 32 or 33 gauge.

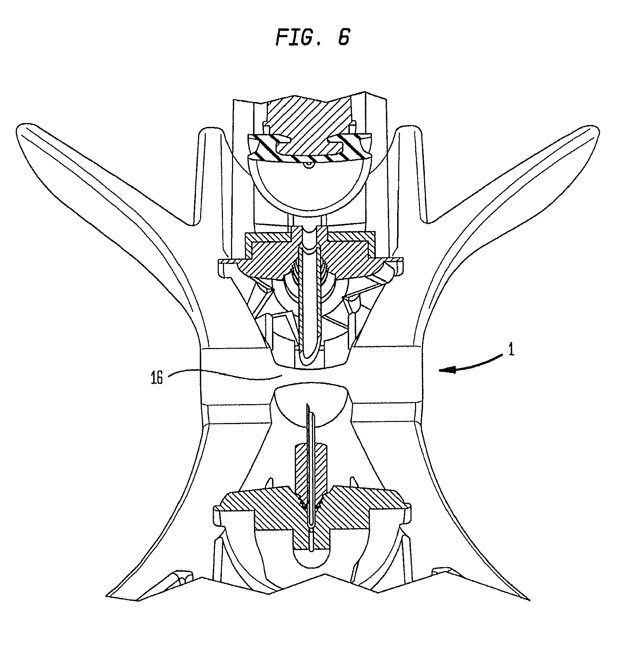

In a related embodiment, the syringe assembly may be pre-loaded with fluid, and the corresponding chamber in the shield apparatus may be empty, ready to receive the larger needle assembly when the user switches to the smaller needle assembly. These embodiments are shown in **Fig. 6**, which shows a shield apparatus that can hold two needle assemblies of different size. The separation 16 between the points of both needles should be sufficient such that the points do not meet, in order to preserve the sterility of any unused needles.

A cross-section of an exemplary double-ended shield apparatuses having chambers for two needles is shown in **Fig. 5**. While **Fig. 5** shows a shield apparatus comprising two chambers of substantially equal size, in certain embodiments the two chambers need not be the same size, and one chamber may hold a larger needle than the other chamber.

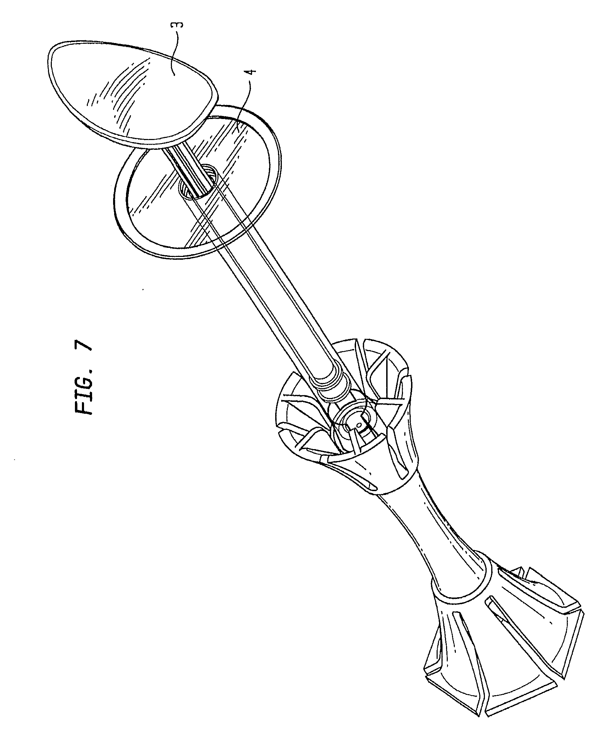

In various embodiments, the syringes of the present invention may include a plunger handle or a syringe flange, both of which may be configured for ease of use - for example, a plunger handle or syringe flange having a loop that can easily be grasped using a single hand, or comprising a soft or pliant material, such as an elastomeric material, 1 that provides a comfortable grip for the user, such as one that yields to the pressure of a user's hand when gripped. An exemplary plunger handle 3 and syringe flange 4 can be seen in one example in **Fig. 7**. At least one may have an ergonomic curved profile, which facilitates the user's ability to use his thumb and index finger to twist the syringe barrel as may be necessary to attach and detach the needle assembly. In certain embodiments, the shield apparatuses of the present invention may further comprise, on their exterior, indentations, protrusions or holes that provide finger positions on which a user can place his fingers, allowing for a firmer grasp during insertion of a syringe assembly into a shield apparatus, as well as removal thereof.

The syringe barrels in the syringe assemblies of the present invention may have fluid volume capacities, in various embodiments, of about 2 to about 5 mL, about 3 mL, about 2 mL, about 1 mL, about 0.5 mL or about 0.3 mL. It has been found that the assemblies of the present invention provide unexpected benefits when used with very small volume barrels and very small (higher gauge) needles.

The shield apparatuses, syringes and needles of the present invention may be made of any materials that are useful for medical devices, including those that are inert, stable, and can be sterilized. Preferably they will not cause undue discomfort or allergic reactions in users or patients. Examples of useful materials are glass, polymeric materials such as plastic (including but not limited to materials comprising polypropylene, polyethylene, polystyrene, polyethylene terephthalate, or low density or high density forms of any of the foregoing), natural or synthetic rubbers, fiberglass, metal and the like.

All embodiments described herein are illustrative and in no way limit the scope of the invention, and the invention may be embodied in other forms not explicitly described here, without departing from the spirit thereof.

## Claims

1. A shield apparatus for storing a needle assembly, the needle assembly comprising a needle fixed to a hub and having a needle point;
wherein the shield apparatus comprises a housing having an exterior wall and an interior wall defining an interior chamber that encloses the needle point;
wherein the shield apparatus is capable of freely standing on a horizontal plane, such that when the shield apparatus stores a syringe assembly, the needle point is facing substantially vertically downward.

2. The shield apparatus of claim 1, wherein the needle assembly is further attached to a syringe barrel.

3. The shield apparatus of claim 2, wherein the needle assembly and syringe barrel are detachably fixed to each other.

4. The shield apparatus of claim 3, wherein the needle assembly and syringe barrel are detachably fixed to each other with a mechanism chosen from: a threading connection, a snap connection and a binary connection.

5. A shield apparatus for needle assemblies, the shield apparatus comprising:
(a) a housing having an exterior wall and an interior wall defining two chambers, each of the two chambers having a length and a width, and each of the two chambers having an open end and a closed end and configured to hold a needle assembly within.

6. The shield apparatus of claim 5, wherein the shield apparatus holds two needle assemblies, the first needle assembly held in the first chamber, and the second needle assembly held in the second chamber; wherein each of the two needle assemblies comprises a needle having a needle point; and wherein each of the two needle assemblies is oriented such that the needle point faces the closed end of each chamber.

7. The shield apparatus of claim 6, wherein the first needle assembly includes a needle having a gauge of 25 or lower.

8. The shield apparatus of claim 6, wherein the second needle assembly includes a needle having a gauge of higher than 25.

9. A syringe assembly comprising:
(a) a cylindrical barrel; and
(b) a needle assembly detachably fixed to the distal end of the cylindrical barrel;
wherein at least a portion of the needle assembly is contained within a shield apparatus.

10. The syringe assembly of claim 9, wherein the shield apparatus is capable of freely standing on a horizontal plane, such that when the at least a portion of the needle assembly is held within the shield apparatus, the needle point is facing substantially vertically downward.

11. The syringe assembly of claim 9, wherein the needle assembly and distal end of the syringe barrel are detachably fixed to each other with a mechanism chosen from: a threading connection, a snap connection and a binary connection.

12. A method of interchanging a first needle assembly and a second needle assembly from a syringe assembly, the method comprising the steps of:
(a) obtaining a syringe assembly including a first needle assembly, wherein the first needle assembly is detachably fixed to the syringe assembly;
(b) inserting the syringe assembly into a first shield apparatus, needle assembly side first;
(c) detaching the first needle assembly from the syringe assembly;
(d) inserting the syringe assembly into a second shield apparatus, the second shield apparatus containing a second needle assembly; and
(c) attaching the second needle assembly to the syringe assembly.

13. The method of claim 12, wherein the first needle assembly includes a needle having a gauge of 25 or lower.

14. The method of claim 13, wherein the first needle assembly includes a needle having a gauge of 18, 20, 22 or 25.

15. The method of claim 12, wherein the second needle assembly includes a needle having a gauge of higher than 25.

16. The method of claim 15, wherein the second needle assembly includes a needle having a gauge of 25-34.

17. A method of administering an injection to a patient, the method comprising the steps of:
(a) obtaining a syringe assembly including a first needle assembly, the first needle assembly including a needle having a gauge of 25 or lower;
(b) loading the syringe assembly with a predetermined amount of fluid;
(c) inserting the syringe assembly into a first shield apparatus, needle assembly side first;
(d) detaching the first needle assembly from the syringe assembly;
(e) inserting the syringe assembly into a second shield apparatus, the second shield apparatus containing a second needle assembly, the second needle assembly including a needle having a gauge of higher than 25; and
(f) attaching the second needle assembly to the syringe assembly.
